# EUROPEAN PATENT SPECIFICATION

(11) **EP 0 512 765 B1**
(45) Date of publication and mention of the grant of the patent: **21.06.1995**
(21) Application number: 92303966.3
(22) Date of filing: 01.05.1992
(51) Int. Cl.: C07C 229/16, C07F 13/00

(54) **Manganese oligomer containing main group elements**
Mangan-Oligomere, die Hauptgruppen-Elemente enthalten
Oligomères de manganèse contenant des éléments de groupes principaux

(30) Priority: 10.05.1991 US 698046
(43) Date of publication of application: 11.11.1992
(73) Proprietor: EXXON RESEARCH AND ENGINEERING COMPANY, Florham Park, New Jersey 07932-0390 (US)
(72) Inventor: Gorun, Sergiu Mircea, Upper Montclair, New Jersey (US); Stibrany, Robert Timothy, Long Valley, New Jersey (US)
(74) Representative: Somers, Harold Arnold

(56) References cited:
- EP-A- 0 466 341
- EP-A- 0 475 695

## Description

This invention relates to novel compositions of matter and their method of preparation. More specifically, this invention relates to novel compounds including 16 manganese ions and a core of 6 barium ions and a carbonate ion.

In co-pending European Application No 91305464.9, EP O,466,341Al there is disclosed a composition of matter having the formula M₂[Mn₄(O)(OH)(O₂CR)₂L₂] wherein M is an alkali earth metal selected from magnesium, calcium, strontium, barium or mixtures thereof, R is hydrogen or a hydrocarbyl group, and L is a ligand having the formula: 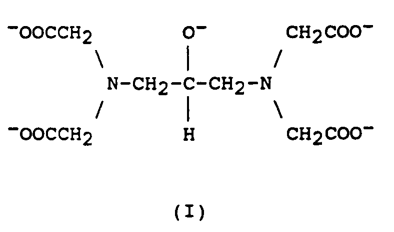
These compounds have been shown to have a core structure of 4 manganese atoms which are bridged by oxo and hydroxo groups and, hence, they are referred to as oxo (hydroxo) bridged tetranuclear manganese compounds.

In co-pending European application No 91308196.4, EP 0,475,695AI, there is disclosed the conversion of the aforementioned oxo (hydroxo) bridged tetranuclear compounds into a hexadecanuclear manganese aggregate having the formula:

[Mn₁₆Ba₈Na₂ClO₄(OH)₄(CO₃)₄(H₂O)₂₂L₈].xH₂O

wherein x is an integer ranging from 0 to about 32 and L is the ligand, (I), shown above.

The present invention is based on the discovery of another hexadecanuclear manganese aggregate having a core of 6 barium ions surrounding a carbonate ion. Stated differently, one aspect of the present invention comprises a composition of matter having the formula:

[Mn₁₆Ba₁₀O₄(OH)₄(CO₃)₅L₈].xH₂0

wherein x is an integer ranging from about 0 to about 120 and L is the ligand, I, shown above.

In another aspect, the invention provides a hexadecanuclear manganese aggregate including a core of 6 barium ions and 5 carbonate ions in which the core is surrounded by 16 manganese ions.

In yet another aspect, the present invention provides a method of preparing the novel compounds of the present invention, which method comprises preparing a chloride free aqueous solution containing a source of carbonate ion selected from sodium carbonate, CO₂ and mixtures thereof and a compound having the formula:

Ba₂[Mn₄(O)(OH)(O₂CR)₂L₂].xH₂0

wherein R is hydrogen or a hydrocarbyl group, x is an integer ranging from 0 to about 30, and L is a ligand having formula I set forth above, and thereafter allowing the reaction mixture to stand for a time sufficient for a compound having the formula:

[Mn₁₆Ba₁₀O₄(OH)₄(CO₃)₅L₈].xH₂O

to form.

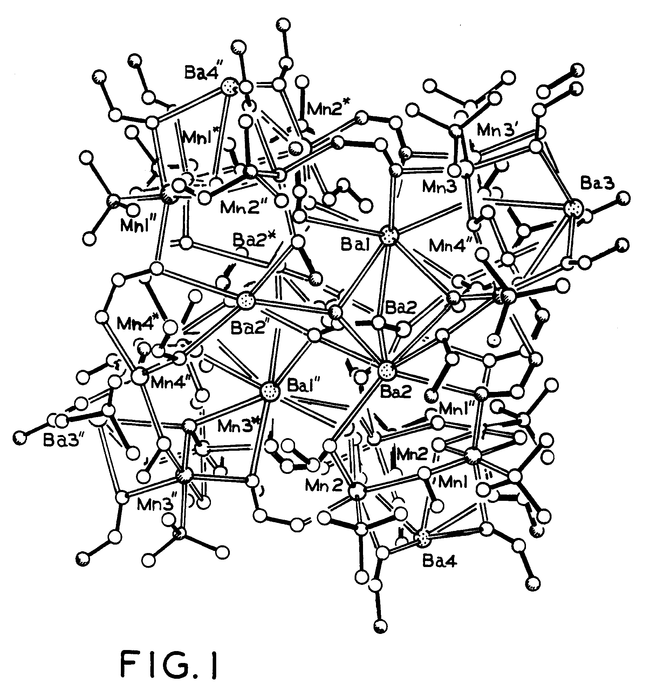

The accompanying Figure 1 shows is a perspective illustration of the structural arrangement of a novel compound of the present invention in which, for purposes of clarity, hydrogen atoms have been omitted; non-hydrogen atoms have been represented by arbitrary-sized spheres; the bonds between Mn, Ba and their coordinated atoms are represented by double lines. Covalent bonds between carbon, hydrogen and oxygen atoms are represented by solid lines.

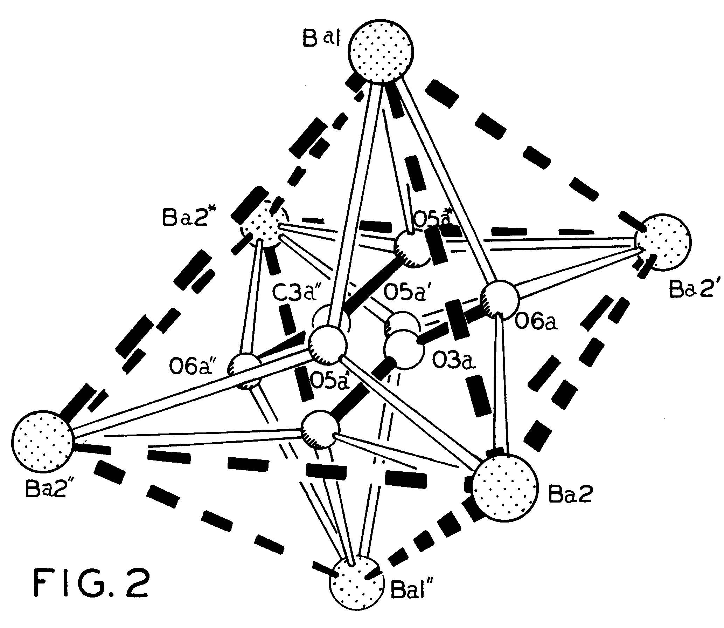

The accompanying Figure 2 shows a perspective illustration of the central [Ba₆CO₃] core of the compound of Figure 1. The 6 barium ions describe an octahedral cage defined by dashed solid lines. The central carbonate ion is disordered over two positions within the barium cage.

The compounds of the present invention have the formula:

[Mn₁₆Ba₁₀O₄(OH)₄(CO₃)₅L₈].xH₂O

wherein x is an integer indicating the amount of water of crystallization, and as such, may vary over a broad range; for example, in the range of 0 to about 120, and L is a ligand having formula I set forth above. Preferably, x is an integer in the range of from about 70 to about 110.

As is shown in Figures 1 and 2, these novel compounds have a core structure of 6 barium ions surrounding a carbonate ion. The barium ions are referred to as main group elements. This main group element core is surrounded by 16 manganese ions.

The structure of the compounds of the present invention has been determined by well-known single crystal x-ray diffraction techniques.
The compounds of the present invention are prepared by combining a chloride free aqueous solution of a source of carbonate ion, such as sodium carbonate or carbon dioxide, and a compound having the formula:

Ba₂[Mn₄(O)(OH)(O₂CR)₂L₂].xH₂O

wherein R is hydrogen or a hydrocarbyl group, especially alkyl, aryl and aralkyl groups and, preferably, R is an alkyl group having from 1 to about 30 carbon atoms. More preferably, R has from 1 to about 10 carbon atoms, and when R is an aralkyl group, it preferably will have from 7 to about 10 carbon atoms. L is a ligand having the formula (I) shown previously, and x is an integer of from 0 to about 30.

The mole ratio of the tetranuclear manganese compound to carbonate source used generally will be in the range of from about 1 to 100 and, preferably, in the range of from about 2 to about 10. When CO₂ is the carbonate source, the CO₂ can be bubbled through the solution for a time sufficient to assure a large excess of CO₂ having been present.

It should be readily appreciated that the tetranuclear compound can be prepared and used in situ and that it is not necessary to first prepare and isolate the tetranuclear compound.

The temperature at which the combined solution is maintained is not critical. Indeed, temperatures of from about 0°C to about 150°C may be used, but it is most convenient and preferred to combine the reactants in water at ambient room temperature, and maintain the mixture at that temperature.

The combined solution is then allowed to stand for a time sufficient for the formation of the desired compound. Typically, crystals of the compounds of the present invention form after the combined solution has been allowed to stand overnight. Alternatively, crystallization can be hastened by known techniques such as reducing the volume of solvent by evaporation, seeding the liquid phase and the like.

The crystalline hexadecanuclear manganese compound is readily separated from the aqueous solution by decantation or filtration. The value of x in the product compound, i.e., the amount of water of hydration, will, of course, depend on the extent of drying of the product. Consequently, x will vary broadly; for example, from about 0 to about 120.

The tetranuclear manganese complex used in preparing the novel compound of the present invention is prepared by combining an aqueous containing solution of the compound having the formula: 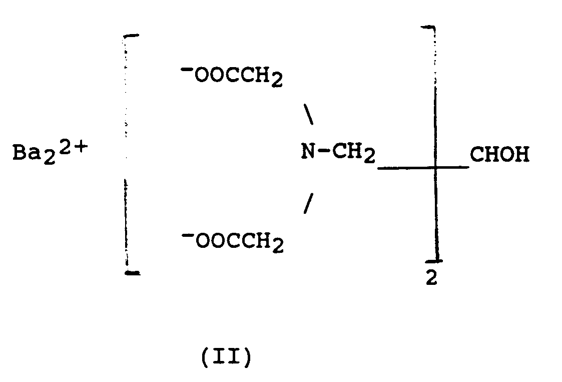
with manganese (II) carboxylate, Mn(O₂CR)₂, or a water-soluble manganese (II) salt and a source of carboxylate RCO₂⁻ in which R is hydrogen or a hydrocarbyl group and thereafter oxidizing the mixture to form the tetranuclear manganese compound. Exemplary hydrocarbyl groups for R include alkyl groups, aryl groups and aralkyl groups, and when R is an alkyl group, it will generally have from about 1 to 30 carbon atoms and, preferably, from 1 to 10 carbon atoms. When R is an aralkyl group, it will generally have from about 7 to about 10 carbon atoms.

Exemplary manganese (II) salts suitable for use in preparing the tetranuclear manganese compound include manganese chloride, manganese bromide, manganese nitrate, manganese tetrafluoroborate and manganese sulfate.

Exemplary sources of carboxylate include carboxylic acids and alkali metal salts of carboxylic acids.

Among suitable aqueous solutions are water, water-alcohol and water-dimethyl formamide mixtures. In general, it is particularly preferred to use water as the solvent in the preparation of the tetranuclear manganese complex.

The mole ratio of the barium compound (formula II above) to manganese (II) carboxylate or manganese (II) salt generally will be in the range of about 1:1 to about 1:3 and, preferably, about 1:2.

The hexadecanuclear manganese compounds of the present invention have a magnetic susceptibility above 100^{o}K, which follows the Curie-Weiss law with ϑ= - 7^{o}K. This magnetic property renders the compounds of the present invention eminently suitable for use in magnetic thermometry and in magnetic fluids.

### EXAMPLES

In the examples which follow, DHPTA refers to 1,3-diamino-2-hydroxypropane-N,N,N'N'-tetraacetic acid, the deprotonated form of which is shown as formula I and is referred to as "L".

### Example 1

In a 50 ml Erlenmeyer flask, 268 mg of DHPTA were added to 20 ml of H₂O and stirred. The pH was brought to 7 with Ba(OH)₂.8H₂O. Then, 430 mg of Mn(OAc)₂.4H₂O were added with 1 ml of methanol and stirred until dissolved. After the pH was brought to 8.0 with Ba(OH)₂.8H₂O, 1 ml of 30% H₂O₂ was added dropwise, giving off heat and gas. The solution was filtered. The filtrate was placed in a 50 ml Erlenmeyer flask, to which 100 mg of Na₂CO₃ were added. Overnight, large green crystals formed, which were characterized by x-ray crystallography and elemental analysis. Calculated for [Mn₁₆Ba₁₀O₄(OH)₄(CO₃)₅L₈].92H₂O: C 16.24%; H 4.28%; N 3.26%. Found: C 15.98%; H 4.21%; N 3.22%.

### Example 2

In a 50 ml Erlenmeyer flask, 268 mg of DHPTA were added to 20 ml of H₂O and the mixture was stirred. The pH was brought to 7 with Ba(OH)₂.8H₂O. Then, 430 mg of Mn(OAc)₂.4H₂O were added with 1 ml of methanol and stirred until dissolved. After the pH was brought to 8.0 with Ba(OH)₂.8H₂O, 1 ml of 30% H₂O₂ was added dropwise, giving off heat and gas. The solution was filtered. The filtrate was placed in a 50 ml Erlenmeyer flask containing 50 mg of solid Ba(OH)₂.8H₂0. TheCO₂ was bubbled into the solution for two hours. Overnight, large green crystals formed, which were identical to those obtained using the procedure in Example 1.

## Claims

1. A composition of matter having the formula:
[Mn₁₆Ba₁₀O₄(OH)₄(CO₃)₅L₈].xH₂O
wherein x is an integer ranging from 0 to about 120 and L is a ligand having the formula:

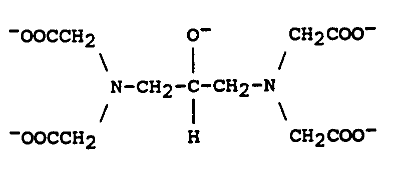

2. The composition of claim 1 wherein x is in the range of from about 70 to about 110.

3. The composition of claim 1 wherein x is 92.

4. The composition of any one of the preceding claims wherein the 16 manganese ions surround a core of the 6 barium ions, which surround a carbonate ion.

5. A hexadecanuclear manganese aggregate including a core of 6 barium ions and 5 carbonate ions in which the core is surrounded by 16 manganese ions.

6. A method for preparing a compound having the formula:
[Mn₁₆Ba₁₀O₄(OH)₄(CO₃)₅L₈].xH₂O
wherein x is an integer of from 0 to about 120 and L is a ligand having the formula: 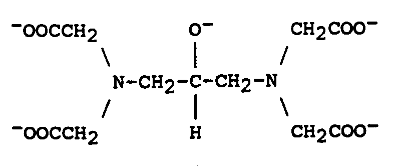 comprising:
forming a chloride free aqueous solution containing a source of CO₃⁼ and a compound having the formula:
Ba₂[Mn₄(O)(OH)(O₂CR)₂L₂].xH₂O
wherein R is hydrogen or a hydrocarbyl group, x is an integer ranging from 0 to about 30, and L is a ligand having the formula above;
allowing the solution to stand for a time sufficient for the compound to form.

7. The method of claim 6 wherein the source of CO₃⁼ is selected from the group consisting of Na₂CO₃, CO₂ and mixtures thereof.

8. The method of claim 6 or claim 7 wherein the mole ratio of Ba₂[Mn₄(O)(OH)(O₂CR)₂L₂].xH₂O to CO₃= is in the range of from about 1:1 to about 100:1.

9. The method of claim 8 wherein the mole ratio of Ba₂[Mn₄(O)(OH)(O₂CR)₂L₂].xH₂O to CO₃= source is in the range of from about 2:1 to about 10:1.

10. The method of any one of claims 6 to 9 wherein the Ba₂[Mn₄(O)(OH)(O₂CR)₂L₂].xH₂O compound is prepared in situ.

11. The method of any one of claims 6 to 9 wherein the Ba₂[Mn₄(O)(OH)(O₂CR)₂L₂].xH₂O is prepared, separated and then combined with the source of CO₃⁼.

## Patentansprüche

1. Materialzusammensetzung mit der Formel:
[Mn₁₆Ba₁₀O₄(OH)₄(CO₃)₅L₈)·xH₂O
, in der x eine ganze Zahl im Bereich von 0 bis etwa 120 und L ein Ligand mit der Formel 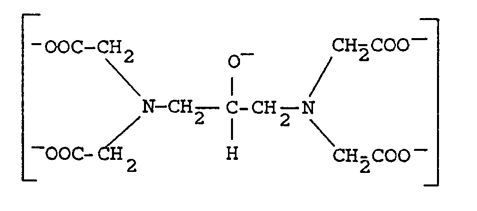 ist.

2. Zusammensetzung nach Anspruch 1, in der x im Bereich von etwa 70 bis etwa 110 liegt.

3. Zusammensetzung nach Anspruch 1, in der x 92 ist.

4. Zusammensetzung nach einem der vorhergehenden Anprüche, in der die 16 Manganionen einen Kern aus 6 Bariumionen umgeben, die ein Carbonation umgeben.

5. Sechzehn-kerniges Mangan-Aggregat, das einen Kern aus 6 Bariumionen und 5 Carbonationen umfaßt, wobei der Kern von 16 Manganionen umgeben ist.

6. Verfahren zur Herstellung einer Verbindung mit der Formel
[Mn₁₆Ba₁₀O₄(OH)₄(CO₃)₅L₈]·xH₂O
, in der x eine ganze Zahl von 0 bis etwa 120 und L ein Ligand mit der Formel 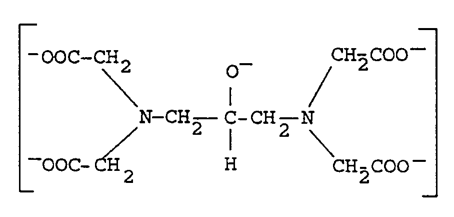 ist, bei dem
eine chlorid-freie wäßrige Lösung gebildet wird, die eine CO₃²⁻-Quelle und eine Verbindung der Formel
Ba₂[Mn₄(O)(OH)(O₂CR)₂L₂]·xH₂O
enthält, in der R Wasserstoff oder eine Kohlenwasserstoffgruppe, x eine ganze Zahl im Bereich von 0 bis etwa 30 und L ein Ligand mit der oben angegebenen Formel ist, und
die Lösung für eine zur Bildung der Verbindung ausreichende Zeit stehengelassen wird.

7. Verfahren nach Anspruch 6, bei dem die CO₃²⁻-Quelle ausgewählt ist aus der Gruppe bestehend aus Na₂CO₃, CO₂ und Mischungen derselben.

8. Verfahren nach Anspruch 6 oder 7, bei dem das Molverhältnis von Ba₂[Mn₄(O)(OH)(O₂CR)₂L₂]·xH₂O zu CO₃²⁻ im Bereich von etwa 1:1 bis etwa 100:1 liegt.

9. Verfahren nach Anspruch 8, bei dem das Molverhältnis von Ba₂[Mn₄(O)(OH)(O₂CR)₂L₂]·xH₂O zu CO₃²⁻ im Bereich von etwa 2:1 bis etwa 10:1 liegt.

10. Verfahren nach einem der Ansprüche 6 bis 9, bei dem die Ba₂[Mn₄(O)(OH)(O₂CR)₂L₂]·xH₂O-Verbindung in situ hergestellt wird.

11. Verfahren nach einem der Ansprüche 6 bis 9, bei dem das Ba₂[Mn₄(O)(OH)(O₂CR)₂L₂]·xH₂O hergestellt, abgetrennt und dann mit der CO₃²⁻-Quelle kombiniert wird.

## Revendications

1. Une composition de matière répondant à la formule
[Mn₁₆Ba₁₀O₄(OH)₄(CO₃)₅L₈].xH₂O
dans laquelle x est un nombre entier s'étendant de 0 à environ 120 et L est un ligand possédant la formule

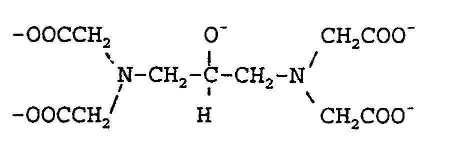

2. La composition selon la revendication 1 dans laquelle x se situe dans la gamme d'environ 70 à environ 110.

3. La composition selon la revendication 1 dans laquelle x est 92.

4. La composition selon l'une quelconque des revendications précédentes dans laquelle les 16 ions manganèse entourent un coeur de 6 ions baryum, qui entourent un ion carbonate.

5. Un agrégat de manganèse hexadécanucléaire comprenant un coeur de 6 ions baryum et de 5 ions carbonate, dans lequel le coeur est entouré de 16 ions manganèse.

6. Un procédé de préparation d'un composé répondant à la formule
[Mn₁₆Ba₁₀O₄(OH)₄(CO₃)₅L₈].xH₂O
dans laquelle x est un nombre entier s'étendant de 0 à environ 120 et L est un ligand possédant la formule 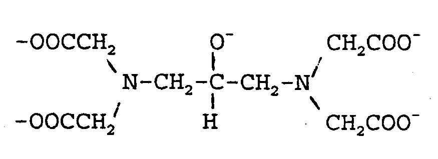 consistant
à former une solution aqueuse exempte de chlorure contenant une source de CO₃⁼ et un composé de formule
Ba₂[Mn₄(O)(OH)(O₂CR)₂L₂].xH₂O
dans laquelle R est l'hydrogène ou un groupe hydrocarbyle, x est un nombre entier s'étendant de 0 à environ 30 et L est un ligand possédant la formule ci-dessus ;
à laisser reposer la solution pendant un temps suffisant pour la formation du composé.

7. Le procédé selon la revendication 6 dans lequel la source de CO₃⁼ est choisie dans le groupe constitué de Na₂CO₃, CO₂ et de leurs mélanges.

8. Le procédé selon la revendication 6 ou la revendication 7 dans lequel le rapport molaire de Ba₂[Mn₄(O)(OH)(O₂CR)₂L₂].xH₂O à CO₃⁼ se situe dans la gamme d'environ 1:1 à environ 100:1.

9. Le procédé selon la revendication 8 dans lequel le rapport molaire de Ba₂[Mn₄(O)(OH)(O₂CR)₂L₂].xH₂O à la source de CO₃⁼ se situe dans la gamme d'environ 2:1 à environ 10:1

10. Le procédé selon l'une quelconque des revendications 6 à 9 dans lequel le composé Ba₂[Mn₄(O)(OH)(O₂CR)₂L₂].xH₂O est préparé in situ.

11. Le procédé selon l'une quelconque des revendications 6 à 9 dans lequel le composé Ba₂[Mn₄(O)(ON)(O₂CR)₂L₂].xH₂O est préparé, séparé et ensuite combiné avec la source de CO₃⁼.
